(19) Europäisches Patentamt
European Patent Office
Office européen des brevets

(11) Veröffentlichungsnummer: **0 173 314**
**B1**

(12) **EUROPÄISCHE PATENTSCHRIFT**

(45) Veröffentlichungstag der Patentschrift: **12.04.89**

(51) Int. Cl.⁴: **C 07 D 285/36**, C 07 D 417/12, A 01 N 43/72 // C07D239/28

(21) Anmeldenummer: **85110825.8**

(22) Anmeldetag: **19.08.85**

(54) **Benzodisultame.**

(30) Priorität: **30.08.84 DE 3431922**
**09.05.85 DE 3516616**

(43) Veröffentlichungstag der Anmeldung: **05.03.86 Patentblatt 86/10**

(45) Bekanntmachung des Hinweises auf die Patenterteilung: **12.04.89 Patentblatt 89/15**

(84) Benannte Vertragsstaaten: **AT BE CH DE FR GB IT LI NL SE**

(56) Entgegenhaltungen:
**EP-A- 0 023 141**
**EP-A- 0 173 316**
**FR-A- 1 571 016**

(73) Patentinhaber: **BAYER AG, Konzernverwaltung RP Patentabteilung, D-5090 Leverkusen 1 Bayerwerk (DE)**

(72) Erfinder: **Diehr, Hans-Joachim, Dr., Höhe 35, D-5600 Wuppertal 1 (DE)**
Erfinder: **Fest, Christa, Dr., Im Johannistal 20, D-5600 Wuppertal 1 (DE)**
Erfinder: **Kirsten, Rolf, Dr., Carl-Langhans-Strasse 27, D-4019 Monheim (DE)**
Erfinder: **Kluth, Joachim, Dr., Kurt-Schumacher-Strasse 9, D-4018 Langenfeld (DE)**
Erfinder: **Müller, Klaus-Helmut, Dr., Bockhackstrasse 55, D-4000 Düsseldorf 13 (DE)**
Erfinder: **Pfister, Theodor, Dr., Lichtenberger Strasse 30, D-4019 Monheim (DE)**
Erfinder: **Priesnitz, Uwe, Dr., Severinstrasse 58, D-5650 Solingen 1 (DE)**
Erfinder: **Riebel, Hans-Jochem, Dr., In der Beek 92, D-5600 Wuppertal 1 (DE)**
Erfinder: **Roy, Wolfgang, Dr., Walter-Kolb-Strasse 47, D-4018 Langenfeld (DE)**
Erfinder: **Santel, Hans-Joachim, Dr., Gerstenkamp 19, D-5000 Köln 80 (DE)**
Erfinder: **Schmidt, Robert R., Dr., Im Waldwinkel 110, D-5060 Bergisch-Gladbach 2 (DE)**

**EP 0 173 314 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neuartige Benzodisultame, welche eine neue Substanzklasse bilden, ein erfinderisches Verfahren zu deren Herstellung und deren Verwendung als Herbizide.

Es ist bereits bekannt, dass bestimmte Benzodisulfonamide herbizide Eigenschaften aufweisen (vgl. EP-A 23 141). Benzodisultame sind dagegen bisher nicht aus der Literatur bekannt.

Es wurden nun neue Benzodisultame der allgemeinen Formel (I)

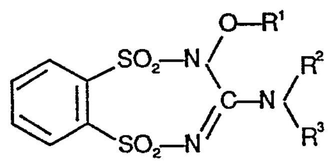

(I)

$R^1$ für $C_1$-$C_{12}$-Alkyl [welches gegebenenfalls durch Fluor, Chlor, Cyano, $C_1$–$C_4$-Alkoxy, $C_1$–$C_4$-Alkylthio, $C_1$–$C_4$-Alkylsulfinyl, $C_1$–$C_4$-Alkylsulfonyl, $C_1$–$C_4$-Alkyl-carbonyl, $C_1$–$C_4$-Alkoxy-carbonyl, $C_1$–$C_4$-Alkylamino-carbonyl oder Di-($C_1$–$C_4$-alkyl)-amino-carbonyl substituiert ist], für $C_3$–$C_6$-Alkenyl (welches gegebenenfalls durch Fluor, Chlor oder Brom substituiert ist), $C_3$–$C_6$-Alkinyl, $C_3$–$C_6$-Cycloalkyl, $C_3$–$C_6$-Cycloalkyl-$C_1$–$C_2$-alkyl, Phenyl-$C_1$–$C_2$-alkyl (welches gegebenenfalls durch Fluor, Chlor, Nitro, Cyano, $C_1$–$C_4$-Alkyl, $C_1$–$C_4$-Alkoxy oder $C_1$–$C_4$-Alkoxy-carbonyl substituiert ist) oder für Phenyl (welches gegebenenfalls durch Fluor, Chlor, Nitro, Cyano, $C_1$–$C_4$-Alkyl, Trifluormethyl, $C_1$–$C_4$-Alkoxy, $C_1$–$C_2$-Fluoralkoxy, $C_1$–$C_4$-Alkylthio, Trifluormethylthio oder $C_1$–$C_4$-Alkoxy-carbonyl substituiert ist) steht, in welcher weiter

$R^2$ für Wasserstoff, $C_1$–$C_4$-Alkyl [welches gegebenenfalls durch Fluor, Chlor, Cyano, $C_1$–$C_4$-Alkoxy, $C_1$–$C_4$-Alkylthio, $C_1$–$C_4$-Alkylsulfinyl, $C_1$–$C_4$-Alkylsulfonyl, $C_1$–$C_4$-Alkyl-carbonyl, $C_1$–$C_4$-Alkoxy-carbonyl, $C_1$–$C_4$-Alkylamino-carbonyl oder Di-($C_1$–$C_4$-alkyl)-amino-carbonyl substituiert ist], für $C_3$–$C_6$-Alkenyl, $C_3$–$C_6$-Alkinyl oderPhenyl-$C_1$–$C_2$-alkyl (welches gegebenenfalls durch Fluor, Chlor, Nitro, Cyano, $C_1$–$C_4$-Alkyl, $C_1$–$C_4$-Alkoxy oder $C_1$–$C_4$-Alkoxy-carbonyl substituiert ist) steht, in welcher ferner

$R^3$ für den Rest

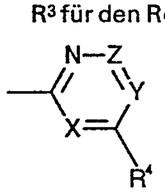

steht, worin

$R^4$ für Wasserstoff, Fluor, Chlor, Brom, Hydroxy, $C_1$–$C_4$-Alkyl (welches gegebenenfalls durch Fluor und/oder Chlor substituiert ist), $C_1$–$C_4$-Alkoxy (welches gegebenenfalls durch Fluor und/oder Chlor substituiert ist), $C_1$–$C_4$-Alkylthio (welches gegebenenfalls durch Fluor und/oder Chlor substituiert ist), Amino, $C_1$–$C_4$-Alkylamino oder Di-($C_1$–$C_4$-alkyl)-amino steht,

X für Stickstoff oder eine Methin-brücke (CH) steht,

Y für Stickstoff oder eine gegebenenfalls substituierte Methin-brücke C-$R^5$ steht, wobei

$R^5$ für Wasserstoff, Fluor, Chlor, Brom, $C_1$–$C_4$-Alkyl, $C_1$–$C_4$-Alkylcarbonyl oder $C_1$–$C_4$-Alkoxycarbonyl steht und

Z für Stickstoff oder eine gegebenenfalls substituierte Methin-brücke C–$R^6$ steht, wobei

$R^6$ für Wasserstoff, Fluor, Chlor, Brom, Hydroxy, $C_1$–$C_4$-Alkyl (welches gegebenenfalls durch Fluor und/oder Chlor substituiert ist), $C_1$–$C_4$-Alkoxy (welches gegebenenfalls durch Fluor und/oder Chlor substituiert ist), $C_1$–$C_4$-Alkylthio (welches gegebenenfalls durch Fluor und/oder Chlor substituiert ist), Amino, $C_1$–$C_4$-Alkylamino oder Di-($C_1$–$C_4$-alkyl)-amino steht.

Man erhält die neuen Verbindungen der Formel (I), wenn man Benzol-1,2-disulfonsäure-dichlorid der Formel (II)

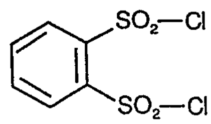

(II)

mit Oxyguanidin-Derivaten der Formel (III)

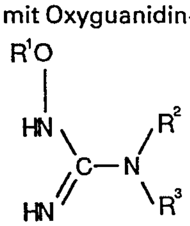

(III)

in welcher

$R^1$, $R^2$ und $R^3$ die oben angegebenen Bedeutungen haben, in Gegenwart von Säureakzeptoren und gegebenenfalls in Gegenwart von Verdünnungsmitteln umsetzt.

Die neuen Benzodisultame der Formel (I) zeichnen sich durch starke herbizide Wirksamkeit aus.

Überraschenderweise zeigen die neuen Verbindungen der Formel (I) erheblich stärkere herbizide Wirkung als viele bekannte chemische Verbindungen gleicher Wirkungsrichtung.

Gegenstand der Erfindung sind vorzugsweise Verbindungen der Formel (I), in welcher

$R^1$ für $C_1$–$C_8$-Alkyl (welches gegebenenfalls durch Fluor oder Chlor substituiert ist), $C_3$–$C_4$-Alkenyl, $C_1$–$C_2$-Alkoxy-carbonylmethyl, Phenyl, Phenylethyl oder Benzyl (welches gegebenenfalls durch Fluor, Chlor, Nitro, Cyano, Methyl, Methoxy oder Methoxy-carbonyl substituiert ist) steht,

$R^2$ für Wasserstoff steht und

$R^3$ für den Rest

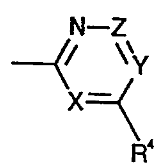

steht, worin

$R^4$ für Chlor, Methyl, Ethyl, Methoxy, Ethoxy, Methylthio oder Difluormethoxy steht,

X für Stickstoff steht,

Y für eine Methin-brücke (CH) steht, und

Z für eine gegebenenfalls substituierte Methinbrücke C-$R^6$ steht, wobei

$R^6$ für Wasserstoff, Chlor, Methyl, Methoxy, Ethoxy, Methylthio, Ethylthio, Dimethylamino oder Diethylamino steht.

Verwendet man beim erfindungsgemässen Verfahren beispielsweise N'-(4,6-Dimethyl-pyrimidin-2-yl)-N''-allyloxy-guanidin und Benzol-1,2-disulfonsäure-dichlorid als Ausgangsstoffe, so kann der Reaktionsablauf durch folgendes Formelschema skizziert werden:

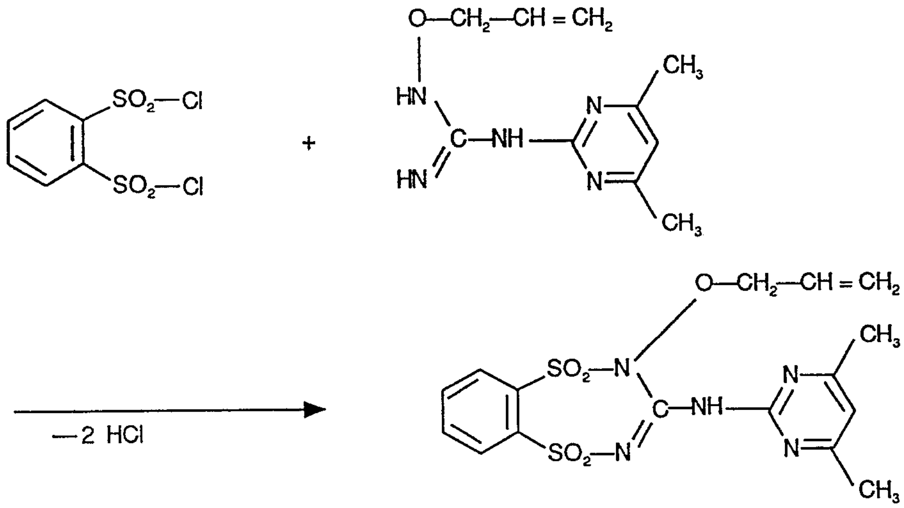


Das beim erfindungsgemässen Verfahren als Ausgangsstoff zu verwendende Benzol-1,2-disulfonsäure-dichlorid der Formel (II) ist bereits bekannt [vergl. J. Org. Chem. 31 (1966), 3289-3292].

Die beim erfindungsgemässen Verfahren weiter als Ausgangsstoffe zu verwendenden Oxyguanidin-Derivate sind durch die Formel (III) allgemein definiert. In Formel (III) haben $R^1$, $R^2$ und $R^3$ vorzugsweise die gleichen Bedeutungen, wie sie oben im Rahmen der Substituentendefinition der Formel (I) vorzugsweise angegeben sind.

Als Ausgangsstoffe der Formel (III) seien beispielsweise genannt:
N'-(4-Methyl-pyrimidin-2-yl)-,
N'-(4,6-Dimethyl-pyrimidin-2-yl)-,
N'-(4-Methoxy-6-methyl-pyrimidin-2-yl)-,
N'-(4-Ethyl-pyrimidin-2-yl)-,
N'-(4-Ethoxy-6-methyl-pyrimidin-2-yl)-,
N'-(4-Chlor-6-methoxy-pyrimidin-2-yl)-,
N'-(4-Chlor-6-ethoxy-pyrimidin-2-yl)-,
N'-(4-Chlor-6-dimethylamino-pyrimidin-2-yl)-,
N'-(4-Methyl-6-methylthio-pyrimidin-2-yl)-,
N'-(4-Dimethylamino-6-methyl-pyrimidin-2-yl)-,
N'-(4,6-Dimethoxy-pyrimidin-2-yl)-,
N'-(4-Difluormethoxy-6-methyl-pyrimidin-2-yl)-,
N'-(2,6-Dimethyl-pyrimidin-4-yl)- und
N'-(2,6-Dimethoxy-pyrimidin-4-yl)-N''-methoxy-guanidin,
-N''-ethoxy-guanidin,
-N''-propoxy-guanidin,
-N''-isopropoxy-guanidin,
-N''-butoxy-guanidin,
-N''-isobutoxy-guanidin,
-N''-sec.-butoxy-guanidin,
-N''-pentoxy-guanidin,
-N''-isopentoxy-guanidin,
-N''-hexyloxy-guanidin,
-N''-octyloxy-guanidin,
-N''-allyloxy-guanidin,
-N''-(2-chlor-ethoxy)-guanidin,
-N''-(2-fluor-ethoxy)-guanidin,
-N''-(2-chlor-propoxy)-guanidin,
-N''-(2-fluor-propoxy)-guanidin,
-N''-(3-chlor-propoxy)-guanidin,
-N''-(4-chlor-butoxy)-guanidin,
-N''-methoxycarbonylmethoxy-guanidin,
-N''-ethoxycarbonylmethoxy-guanidin,
-N''-(1-methoxy-carbonyl-ethoxy)-guanidin,
-N''-(1-ethoxy-carbonyl-ethoxy)-guanidin,
-N''-(dimethylamino-carbonyl-methoxy)-guanidin,
-N''-(2-phenyl-ethoxy)-guanidin,
-N''-phenoxy-guanidin,
-N''-(4-methyl-benzyloxy)-guanidin,
-N''-(4-fluor-benzyloxy)-guanidin,
-N''-(4-chlor-benzyloxy)-guanidin,
-N''-(4-nitro-benzyloxy)-guanidin,
-N''-(2,6-dichlor-benzyloxy)-guanidin,
-N''-(4-methoxycarbonyl-benzyloxy)-guanidin und
-N''-(4-ethoxycarbonyl-benzyloxy)-guanidin.

Die Ausgangsstoffe der Formel (III) sind zum Teil bekannt (vergl. J. Chem. Soc. 1962, 3915 und EP-A 121 082).

Man erhält die Verbindungen der Formel (III) wenn man Cyanamid-Derivate der Formel (IV)

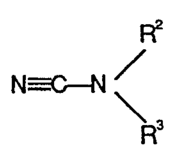 (IV)

in welcher

$R^2$ und $R^3$ die oben angegebenen Bedeutungen

haben, mit Hydroxylamin-Derivaten der Formel (V)

$H_2N-OR^1$ (V)

in welcher

$R^1$ die oben angegebene Bedeutung hat, bzw. deren Hydrochloriden, gegebenenfalls in Gegenwart von Verdünnungsmitteln, wie z.B. Ethanol, Propanol oder Butanol, bei Temperaturen zwischen 20°C und 120°C umsetzt und gegebenenfalls die Umsetzungsprodukte mit Säureakzeptoren, wie z.B. Ammoniak, Kaliumcarbonat oder Natriumhydroxid, behandelt. Die Cyanamid-Derivate der Formel (IV) sind zum Teil bekannt (vergl. J. Chem. Soc. 1953, 1725). Man erhält die Verbindungen der Formel (IV) im wesentlichen nach folgenden Synthesewegen:

(a) durch Umsetzung von Alkalimetall- oder Erdalkalimetall-Salzen von Cyanamid – wie z.B. Natriumcyanamid oder Calciumcyanamid – mit Chlor-hetarenen der Formel (VI)

$Cl-R^3$ (VI)

in welcher

$R^3$ die oben angegebene Bedeutung hat, und gegebenenfalls anschliessend – wenn $R^2$ nicht für Wasserstoff steht – mit Halogenverbindungen der Formel (VII)

$Q-R^2$ (VII)

in welcher

$R^2$ für einen gegebenenfalls substituierten Rest aus der Reihe Alkyl, Alkenyl, Alkinyl und Aralkyl steht, und

Q für Chlor, Brom oder Iod steht, gegebenenfalls in Gegenwart von inerten Verdünnungsmitteln, wie z.B. Aceton, Acetonitril oder Dimethylformamid, bei Temperaturen zwischen 0°C und 100°C.

Nach Einengen und Auflösen des Rückstandes in Wasser können die Cyanamid-Derivate der Formel (IV) durch Ansäuern, z.B. mit Salzsäure, ausgefällt und durch Absaugen isoliert werden.

Alternativ erhält man die Verbindungen der Formel (IV)

(b) für den Fall, dass $R^3$ für einen substituierten Pyrimidinylrest steht, durch Umsetzung von Cyanoguanidin («Dicyandiamid») mit β-Dicarbonylverbindungen oder deren Derivaten, wie z.B. Acetylaceton (vergl. J. Chem. Soc. 1953, 1725–1730), Acetessigsäureestern [vergl. J. Prakt. Chem. 77 (1908), 542 und J. Chem. Soc. 1948, 586] oder Malonsäureestern (vergl. DE-PS 158 591).

Die aus Acetessigsäureestern bzw. Malonsäureestern erhaltenen 2-Cyanamino-4-hydroxy-6-methyl- bzw. -4,6-dihydroxy-pyrimidine können auf bekannte Weise durch Umsetzung mit Alkylierungsmitteln, wie z.B. Dimethyl- oder Diethylsulfat, gegebenenfalls in Gegenwart von Verdünnungsmitteln, wie z.B. Wasser, Methanol, Ethanol, n- und iso-Propanol, Aceton, Dioxan oder Dimethylformamid, und in Gegenwart von Säurebindemitteln, wie z.B. Natrium- oder Kalium-hydroxid, Natrium- oder Kalium-carbonat, in entsprechende 2-Cyanamino-4-alkoxy-6-methyl- bzw. -4,6-dialkoxy-pyrimidine umgewandelt werden. Zur Vermeidung einer N-Alkylierung wird gegebenenfalls mit einem Acylierungsmittel, wie z.B. Acetanhydrid oder Acetylchlorid acyliert und nach der Alkylierung mit wässrigen Säuren oder Basen wieder entacyliert.

In einem weiteren Alternativverfahren erhält man die Verbindungen der Formel (IV), wenn man

(c) Amino-hetarene der Formel (VIII)

$H_2N-R^3$ (VIII)

in welcher

$R^3$ die oben angegebene Bedeutung hat, mit Carbonylisothiocyanaten der Formel (IX)

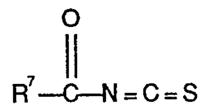 (IX)

in welcher

$R^7$ für Ethoxy oder Phenyl steht, gegebenenfalls in Gegenwart eines inerten Verdünnungsmittels, wie z.B. Aceton, Acetonitril oder Toluol, bei Temperaturen zwischen 0°C und 100°C umsetzt, die hierbei gebildeten Carbonylthioharnstoffe der Formel (X)

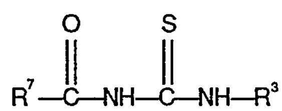 (X)

in welcher

$R^3$ und $R^7$ die oben angegebenen Bedeutungen haben, gegebenenfalls nach Einengen durch Absaugen isoliert und mit wässrigen Alkalimetall- oder Erdalkalimetall-hydroxidlösungen, wie z.B. Natronlauge, gegebenenfalls in Gegenwart eines organischen Lösungsmittels, wie z.B. Tetrahydrofuran oder Dioxan, bei Temperaturen zwischen 0°C und 120°C umsetzt und die nach Ansäuern, z.B. mit Salzsäure, kristallin erhaltenen Thioharnstoffe der Formel (XI)

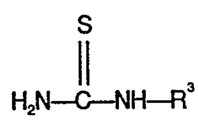 (XI)

in welcher

$R^3$ die oben angegebene Bedeutung hat, durch Absaugen isoliert und mit Metallverbindungen, welche Schwefelwasserstoff binden können, wie z.B. mit Blei(II)-acetat, Kupfer(II)-acetat, Quecksilber(II)-acetat oder Eisen(II)-acetat, in Gegenwart von wässrigen Alkalimetall- oder Erdalkalimetall-hydroxidlösungen, wie z.B. Natronlauge, bei Temperaturen zwischen 20°C und 100°C umsetzt, nach Ende der Umsetzung filtriert und das Filtrat mit einer Säure, wie z.B. Essigsäure, ansäuert. Die hierbei kristallin anfallenden Produkte

der Formel (IV) können durch Absaugen isoliert werden.

Die Ausgangsstoffe für die vorausgehend unter (a), (b) und (c) beschriebenen Herstellungsverfahren für die Cyanamid-Derivate der Formel (IV) sind bekannt und/oder können nach an sich bekannten Verfahren hergestellt werden.

Hierzu gehören die Chlor-hetarene der Formel (VI) [vergl. J. Chem. Soc. (C) 1966, 2031; Chem. Pharm. Bull. 11 (1963), 1382-1388 und Arch. Pharm. 295 (1962), 649-657], die Halogenverbindungen der Formel (VII) (handelsübliche Chemikalien), die Amino-hetarene der Formel (VIII) [vergl. Chem. Pharm. Bull. 11 (1963), 1382-1388; J. Chem. Soc. 1946, 81 und US-PS 4 299 960] und die Carbonylisothiocyanate der Formel (IX) [vergl. J. Heterocycl. Chem. 5 (1968), 837 und US-PS 4 160 037].

Das erfindungsgemässe Verfahren zur Herstellung der neuen Verbindungen der Formel (I) wird vorzugsweise unter Verwendung von Verdünnungsmitteln durchgeführt. Als solche kommen praktisch alle inerten organischen, vorzugsweise jedoch aprotisch polare Solventien in Betracht. Hierzu gehören gegebenenfalls halogenierte Kohlenwasserstoffe, wie z.B. Methylenchlorid, Chloroform, Toluol und Chlorbenzol, Nitrile, wie z.B. Acetonitril und Propionsäurenitril, Dimethylformamid, Dimethylacetamid, Dimethylsulfoxid, Sulfolan, Hexamethylphosphorsäuretriamid, 1,2-Dimethoxyethan, Pyridin und 2-Methyl-5-ethylpyridin.

Als Säureakzeptoren können beim erfindungsgemässen Verfahren praktisch alle üblicherweise verwendeten Säurebindemittel eingesetzt werden. Hierzu gehören insbesondere Alkalimetall- und Erdalkalimetall-hydride, metallorganische Verbindungen, wie Butyllithium, ferner aliphatische, aromatische oder heterocyclische Amine, wie Trimethylamin, Triethylamin, N,N-Dimethylanilin, N,N-Dimethyl-benzylamin, Diazabicyclooctan (DABCO), Diazabicycloundecen (DBU), Pyridin, 2-Methyl-5-ethyl-pyridin und 4-Dimethylamino-pyridin.

Die Reaktionstemperaturen können beim erfindungsgemässen Verfahren innerhalb eines grösseren Bereichs variiert werden. Im allgemeinen arbeitet man zwischen −80°C und +100°C, vorzugsweise zwischen −40°C und +50°C. Das erfindungsgemässe Verfahren wird im allgemeinen bei Normaldruck durchgeführt.

Zur Durchführung des erfindungsgemässen Verfahrens setzt man je Mol Oxyguanidin-Derivat der Formel (III) im allgemeinen zwischen 1,0 und 1,5 Mol, vorzugsweise zwischen 1,0 und 1,2 Mol Benzol-1,2-disulfonsäure-dichlorid der Formel (II) ein. Die Reaktionskomponenten werden gewöhnlich bei Raumtemperatur oder unter Aussenkühlung zusammengegeben und das Reaktionsgemisch wird bis zum Reaktionsende gerührt.

Die Aufarbeitung und Isolierung der neuen Verbindungen der Formel (I) kann nach üblichen Methoden durchgeführt werden. Beispielsweise wird das Reaktionsgemisch - gegebenenfalls nach Verdünnen mit einem mit Wasser praktisch nicht mischbaren Lösungsmittel, wie z.B. Methylenchlorid - mit verdünnter Salzsäure und mit Wasser gewaschen, getrocknet, filtriert und eingeengt. Das im Rückstand verbliebene Produkt der Formel (I) wird durch Verreiben mit einem geeigneten organischen Lösungsmittel, wie z.B. Ethanol zur Kristallisation gebracht und durch Absaugen isoliert.

Die erfindungsgemässen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemässen Stoffe als totale oder selektive Herbizide wirken, hängt im wesentlichen von der angewendeten Menge ab.

Die erfindungsgemässen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cuburbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monocharia, Fimbristylis, Sagittaria, Eleocharis, Scirpus, Paspalum, Ischaemum, Spenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemässen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemässen Wirkstoffe eignen sich zur Bekämpfung mono- und dikotyler Unkräuter in mono- und dikotylen Kulturen im Vor-und im Nachauflaufverfahren. Eine Kontrolle keimender, auflaufender und bereits etablierter Unkräuter in Dauerkulturen ist ebenso möglich, wie die totale Vegetationsbekämpfung auf Nichtkulturland.

Die Wirkstoffe können in die üblichen Formulierungen überführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweisshydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%. Die Formulierungen haben einen Gehalt an mindestens einem erfindungsgemässen Wirkstoff der Formel (I).

Die erfindungsgemässen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. N-(2-Benzthiazolyl)-N,N'-dimethylharnstoff, 3-(3-Chlor-4-methylphenyl)-1,1-dimethylharnstoff, 3-(4-Isopropylphenyl)-1,1-dimethylharnstoff, 4-Amino-6-(1,1-dimethylethyl)-3-methylthio-1,2,4-triazin-5-(4H)-on, 4-Amino-6-(1,1-dimethyl-ethyl)-3-ethylthio-1,2,4-triazin-5-(4H)-on, 1-Amino-6-ethylthio-3-(2,2-dimethylpropyl)-1,3,5-triazin-2,4-(1H,3H)-dion, 4-Amino-3-methyl-6-phenyl-1,2,4-triazin-5-(4H)-on, 2-Chlor-4-ethylamino-6-isopropylamino-1,3,5-triazin, das R-Enantiomere des 2-[4-(3,5-Dichlor-pyridyl-2-oxy)-phenoxy]-propionsäure-(trimethylsilyl)-methylesters, das R-Enantiomere des 2-[4-(3,5-Dichlorpyridyl-2-oxy)-phenoxy]-propionsäure-(2-benzyloxy)-ethylesters, 2,4-Dichlorphenoxyessigsäure, 2-(2,4-Dichlorphenoxy)-propionsäure, 4-Chlor-2-methylphenoxyessigsäure, 2-(2-Methyl-4-chlor-phenoxy)-prioponsäure, 3,5-Diiod-4-hydroxy-benzonitril, 3,5-Dibrom-4-hydroxy-benzonitril sowie Diphenylether und Phenylpyridazine, wie z.B. Pyridate. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfrass, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Giessen, Spritzen, Sprühen, Streuen.

Die erfindungsgemässen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in

einem grösseren Bereich schwanken. Sie hängt im wesentlichen von der Art des gewünschten Effektes ab. Im allgemeinen liegen die Aufwandmengen zwischen 0,001 und 15 kg Wirkstoff pro Hektar Bodenfläche, vorzugsweise zwischen 0,005 und 10 kg pro ha.

Die Herstellung und die Verwendung der erfindungsgemässen Wirkstoffe geht aus den nachfolgenden Beispielen hervor.

Herstellungsbeispiele

Beispiel 1

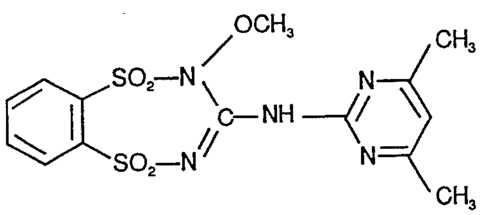

14 g (0,05 Mol) Benzol-1,2-disulfonsäure-dichlorid werden portionsweise bei −20 °C zu einer Mischung von 10 g (0,05 Mol) N′-(4,6-Dimethyl-pyrimidin-2-yl)-N″-methoxyguanidin, 12 g (0,15 Mol) Pyridin und 100 ml Methylenchlorid gegeben. Man rührt 3 Stunden bei −20 °C und 15 Stunden bei +20 °C nach. Dann wird das Reaktionsgemisch mit eisgekühlter verdünnter Salzsäure und Eiswasser gewaschen. Die Methylenchloridlösung wird getrocknet und eingeengt. Der Rückstand wird mit Ethanol verrieben. Der hierbei kristallin angefallene Rückstand wird durch Absaugen isoliert.

Man erhält 11,5 g (58% der Theorie) der Verbindung der oben angegebenen Strukturformel vom Schmelzpunkt 158 °C (Zers.).

Die Aufarbeitung des Reaktionsgemisches kann auch in der Weise erfolgen, dass man nach beendeter Reaktion vollständig eindampft, den Rückstand in Dioxan aufnimmt, filtriert, erneut eindampft und den Rückstand umkristallisiert.

Nach dem im vorausgehenden Beispiel exemplarisch beschriebenen Verfahren können die in der nachstehenden Tabelle 1 aufgeführten Verbindungen der Formel (I) hergestellt werden:

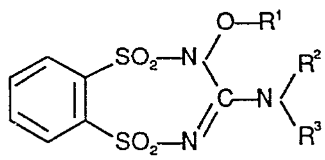

(I)

Tabelle 1

| Beisp. Nr. | $R^1$ | $R^2$ | $R^3$ | Schmelzpunkt (°C) |
|---|---|---|---|---|
| 2 | $-C_2H_5$ | H | 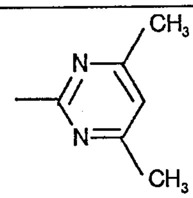 | 104 |
| 3 | $-C_3H_7$(-i) | H | 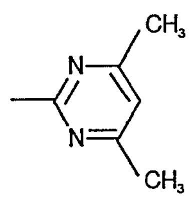 | (amorph) |
| 4 | $-C_3H_7$(-n) | H | 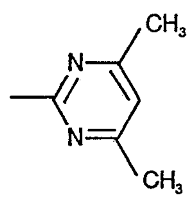 | 134 |
| 5 | $-C_4H_9$(-n) | H | 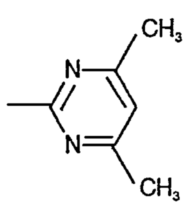 | 179 (Zers.) |

Tabelle 1 (Fortsetzung)

| Beisp. Nr. | R¹ | R² | R³ | Schmelzpunkt (°C) |
|---|---|---|---|---|
| 6 | $-C_8H_{17}$(-n) | H | 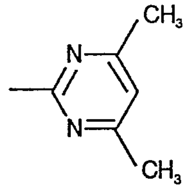 | 164 |
| 7 | 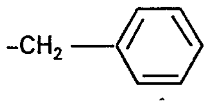 | H | 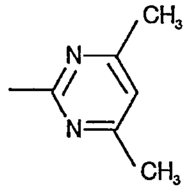 | 198 |
| 8 | 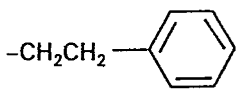 | H | 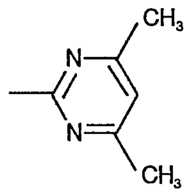 | |
| 9 | $-CH_2COOC_2H_5$ | H | 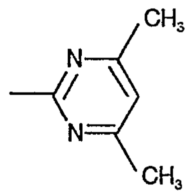 | 210 (Zers.) |
| 10 | $-C_4H_9$(-i) | H | 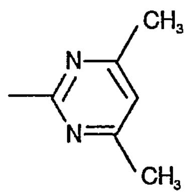 | |
| 11 | $-C_4H_9$(-sec.) | H | 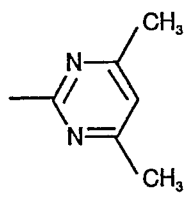 | |
| 12 | 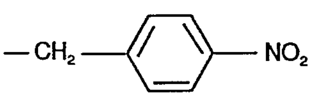 | H | 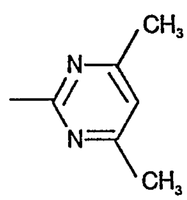 | |

Tabelle 1 (Fortsetzung)

| Beisp. Nr. | $R^1$ | $R^2$ | $R^3$ | Schmelzpunkt (°C) |
|---|---|---|---|---|
| 13 | 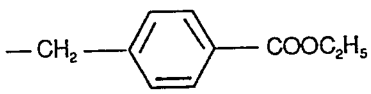 | H | 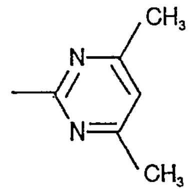 | |
| 14 | 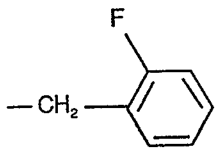 | H | 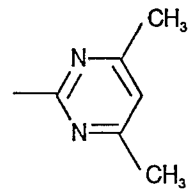 | |
| 15 | 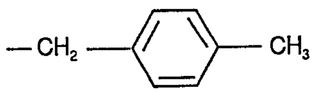 | H | 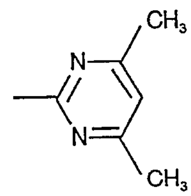 | |
| 16 | 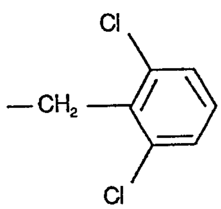 | H | 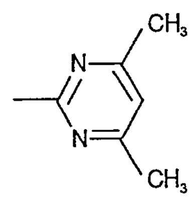 | |
| 17 | $-CH_3$ | $-CH_3$ | 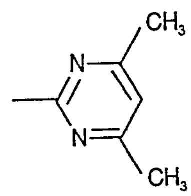 | |
| 18 | $-CH_2-CH=CH_2$ | H | 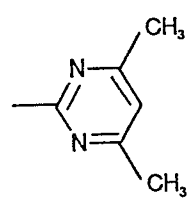 | 180 |
| 19 | $-CH_3$ | H | 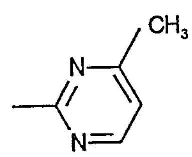 | |

Tabelle 1 (Fortsetzung)

| Beisp. Nr. | $R^1$ | $R^2$ | $R^3$ | Schmelzpunkt (°C) |
|---|---|---|---|---|
| 20 | $-C_2H_5$ | H | 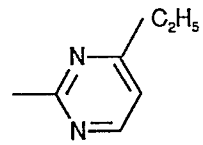 | |
| 21 | $-CH_3$ | H | 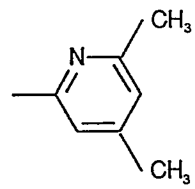 | |
| 22 | $-CH_3$ | H | 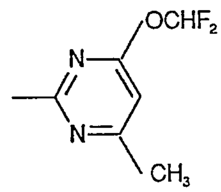 | |
| 23 | $-CH_3$ | H | 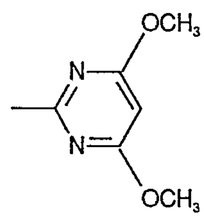 | |
| 24 | $-C_4H_9$(-sec.) | H | 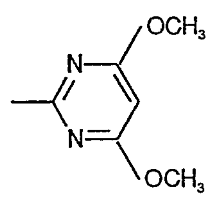 | |
| 25 | $-C_4H_9$(-i) | H | 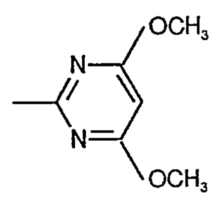 | |
| 26 | $-CH_3$ | H | 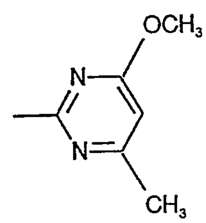 | 151 (Zers.) |

Tabelle 1 (Fortsetzung)

| Beisp. Nr. | $R^1$ | $R^2$ | $R^3$ | Schmelzpunkt (°C) |
|---|---|---|---|---|
| 27 | $-CH_3$ | $-CH_3$ | 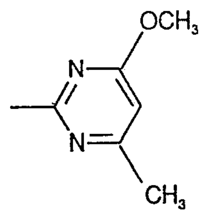 | 135 |
| 28 | $-CH_3$ | H | 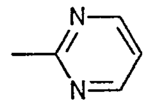 | 187 |

Herstellung von Ausgangsstoffen der Formel (III)

Beispiel (III-1)

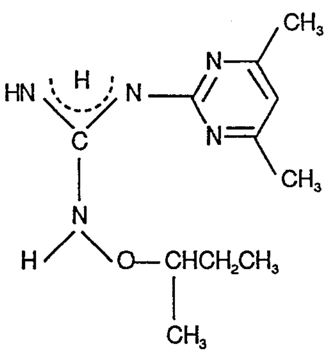

Eine Mischung aus 143 g (0,97 Mol) 2-Cyanamino-4,6-dimethyl-pyrimidin, 94 g (1,06 Mol) 0-sec.-Butyl-hydroxylamin und 190 ml Ethanol wird 6 Stunden unter Rückfluss zum Sieden erhitzt. Dann wird abgesaugt, das Filtrat eingeengt und der Rückstand mit 500 ml Wasser versetzt. Das hierbei kristallin angefallene Produkt wird durch Absaugen isoliert.

Man erhält 131 g (57% der Theorie) N'-(4,6-Dimethyl-pyrimidin-2-yl)-N''-sec.-butoxy-guanidin vom Schmelzpunkt 78 °C.

Analog können die in der nachstehenden Tabelle 2 aufgeführten Verbindungen der Formel (III) hergestellt werden:

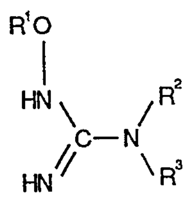

(III)

Tabelle 2

| Beisp. Nr. | $R^1$ | $R^2$ | $R^3$ | Schmelzpunkt (°C) |
|---|---|---|---|---|
| III-2 | $-CH_2CH(CH_3)_2$ | H | 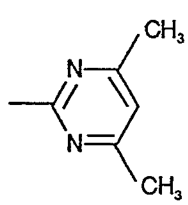 | 52 |
| III-3 | $-CH_2CH=CH_2$ | H | 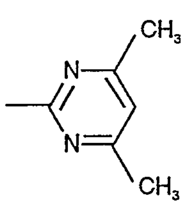 | 103 |

Tabelle 2 (Fortsetzung)

| Beisp.-Nr. | R¹ | R² | R³ | Schmelzpunkt (°C) |
|---|---|---|---|---|
| III-4 | $-CH(CH_3)_2$ | H | 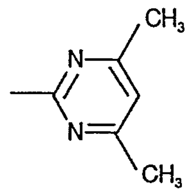 | 84 |
| III-5 | $-CH_2-CH_2-$ 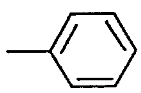 | H | 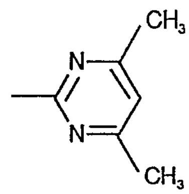 | $n_D^{24} = 1,5776$ |
| III-6 | $-C_4H_9(-n)$ | H | 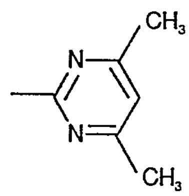 | (Öl) |
| III-7 | $-C_8H_{17}(-n)$ | H | 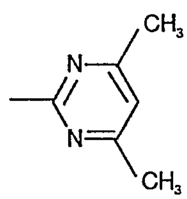 | 58 |
| III-8 | $-CH_2-$ 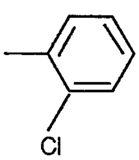 | H | 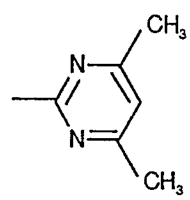 | 102–103 |
| III-9 | $-CH_2CH_2CH_2Cl$ | H | 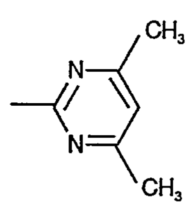 | 137 |
| III-10 | 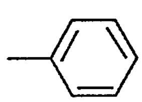 | H | 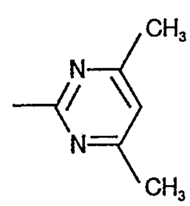 | 189–192 (Zers.) |

Tabelle 2 (Fortsetzung)

| Beisp.-Nr. | $R^1$ | $R^2$ | $R^3$ | Schmelzpunkt (°C) |
|---|---|---|---|---|
| III-11 | $-CH_2COOCH_3$ | H | 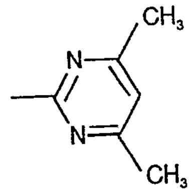 | 148–149 |
| III-12 | $-CH_2COOC_2H_5$ | H | 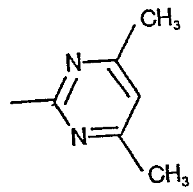 | 98–99 |
| III-13 | 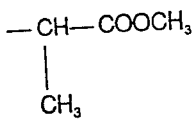 | H | 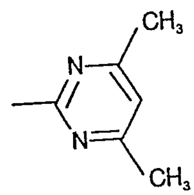 | 147–148 |
| III-14 | 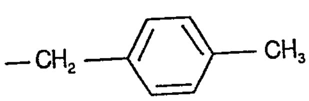 | H | 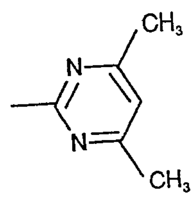 | 85–86 |
| III-15 | 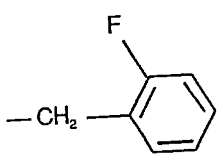 | H | 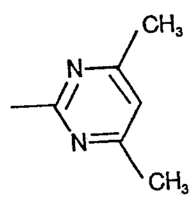 | 114 |
| III-16 | 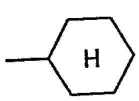 | H | 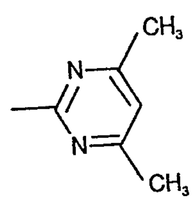 | |
| III-17 | 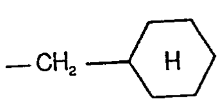 | H | 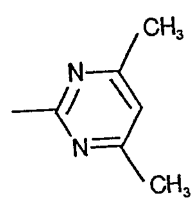 | |

Tabelle 2 (Fortsetzung)

| Beisp.-Nr. | $R^1$ | $R^2$ | $R^3$ | Schmelz-punkt (°C) |
|---|---|---|---|---|
| III-18 | $-CH_2CON(CH_3)_2$ | H | 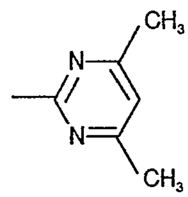 | |
| III-19 | $-CH_2OCH_3$ | H | 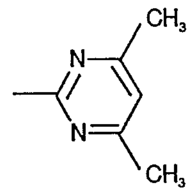 | |
| III-20 | $-CH_2SCH_3$ | H | 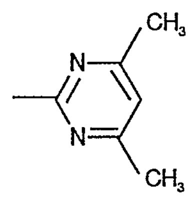 | |
| III-21 | 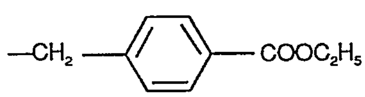 | H | 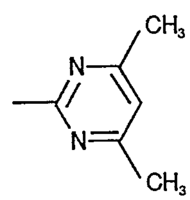 | 138 |
| III-22 | $-CH_2CF_3$ | H | 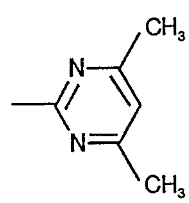 | |
| III-23 | 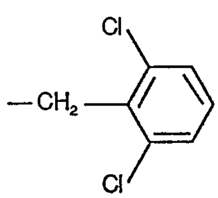 | H | 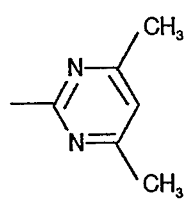 | 140-145 |
| III-24 | 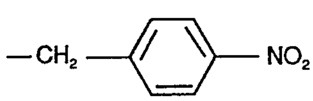 | H | 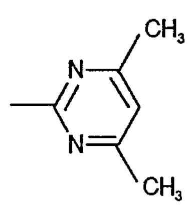 | 170 |

Tabelle 2 (Fortsetzung)

| Beisp.-Nr. | $R^1$ | $R^2$ | $R^3$ | Schmelzpunkt (°C) |
|---|---|---|---|---|
| III-25 | $-CH_3$ | H | 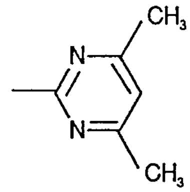 | 134–136 |
| III-26 | $-C_2H_5$ | H | 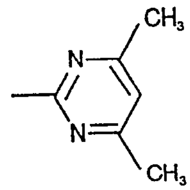 | 88 |
| III-27 | 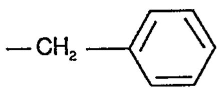 | H | 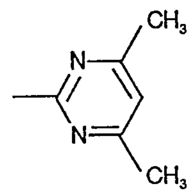 | 102 |
| III-28 | $-CH_3$ | $-CH_3$ | 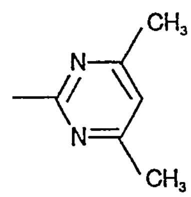 | 95 |
| III-29 | $-CH_3$ | $-CH_3$ | 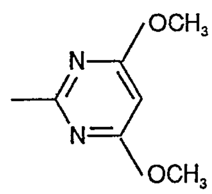 | 135 |
| III-30 | $-CH_3$ | H | 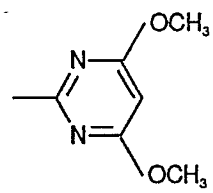 | 122 |
| III-31 | $-CH_3$ | H | 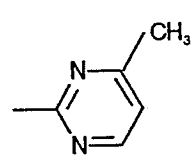 | 152 |

Tabelle 2 (Fortsetzung)

| Beisp.-Nr. | $R^1$ | $R^2$ | $R^3$ | Schmelz-punkt (°C) |
|---|---|---|---|---|
| III-32 | $-CH_3$ | H | 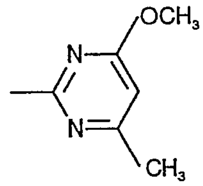 | 126 |
| III-33 | $-CH_3$ | H | 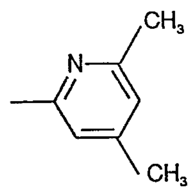 | 110 |
| III-34 | $-CH_3$ | H | 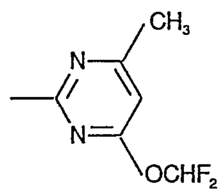 | |
| III-35 | $-C_2H_5$ | H | 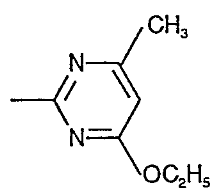 | |
| III-36 | $-C_4H_9$(-sec.) | H | 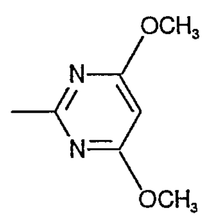 | 68 |
| III-37 | $-C_4H_9$(-i) | H | 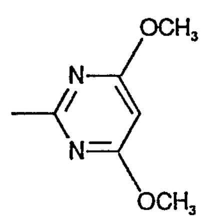 | 76 |
| III-38 | $-CH_3$ | H | 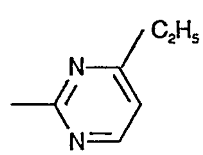 | 98 |

Tabelle 2 (Fortsetzung)

| Beisp.-Nr. | R¹ | R² | R³ | Schmelzpunkt (°C) |
|---|---|---|---|---|
| III-39 | $-C_3H_7(-n)$ | H | 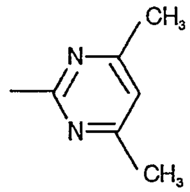 | 54 |
| III-40 | $-CH_2-COOC_3H_7(-i)$ | H | 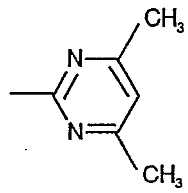 | 112 |
| III-41 | $-C_2H_5$ | H | 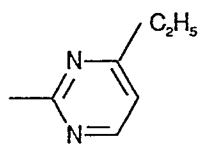 | |
| III-42 | 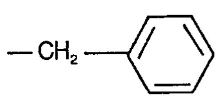 | H | 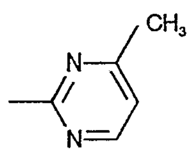 | 150 |
| III-43 | 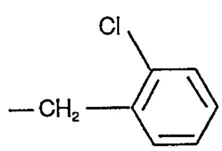 | H | 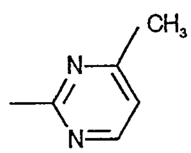 | 140 |
| III-44 | $-C_2H_5$ | H | 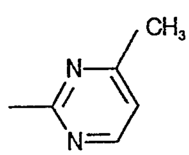 | 95 |
| III-45 | 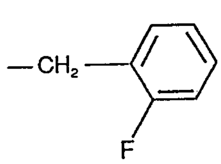 | H | 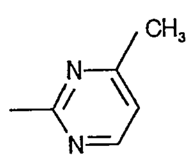 | 205 |

Tabelle 2 (Fortsetzung)

| Beisp.-Nr. | $R^1$ | $R^2$ | $R^3$ | Schmelz-punkt (°C) |
|---|---|---|---|---|
| III-46 | $-CH_2CH_2CH_2Cl$ | H | 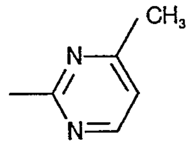 | 102 |
| III-47 | $-CH_2-COOC_2H_5$ | H | 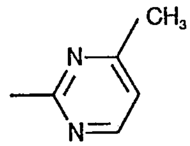 | |
| III-48 | $-CH_2-CH=CH_2$ | H | 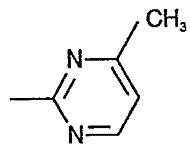 | |
| III-49 | $-C_4H_9(-n)$ | H | 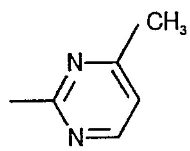 | |
| III-50 | $-C_4H_9(-sec.)$ | H | 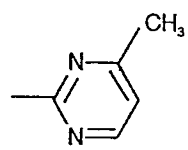 | |
| III-51 | $-CH_3$ | H | 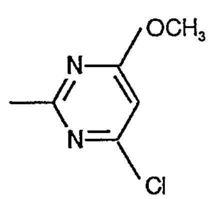 | 112 |
| III-52 | 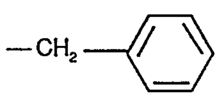 | H | 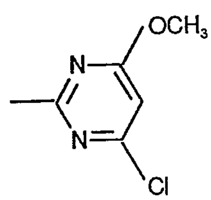 | |

Tabelle 2 (Fortsetzung)

| Beisp.-Nr. | $R^1$ | $R^2$ | $R^3$ | Schmelzpunkt (°C) |
|---|---|---|---|---|
| III-53 | $-CH_3$ | H | 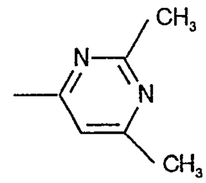 | 143 |
| III-54 | 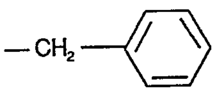 | H | 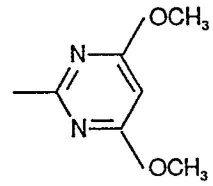 | 74 |
| III-55 | $-CH_3$ | H | 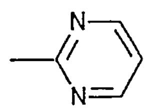 | 107-109 |
| III-56 | $-CH_3$ | H | 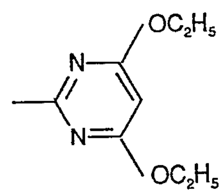 | |
| III-57 | 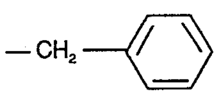 | H | 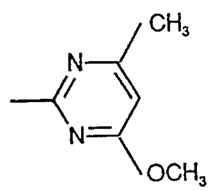 | $n_D^{20} = 1,5645$ |
| III-58 | 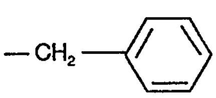 | H | 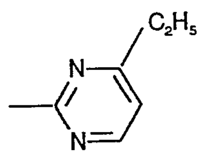 | 112 |
| III-59 | $-CH_2CH_2CH_2CH_2Cl$ | H | 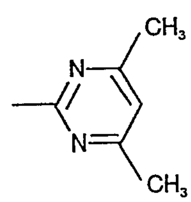 | amorph |

Tabelle 2 (Fortsetzung)

| Beisp.-Nr. | $R^1$ | $R^2$ | $R^3$ | Schmelz-punkt (°C) |
|---|---|---|---|---|
| III-60 | $-CH_2CH_2Cl$ | H | 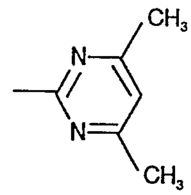 | amorph |
| III-61 | 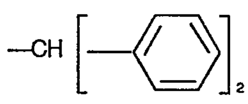 | H | 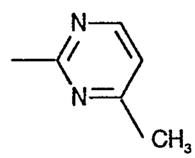 | 165 |
| III-62 | $-CH_2CH=CHCl$ | H | 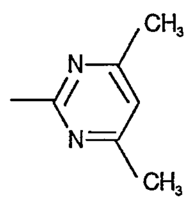 | |

Herstellung der Ausgangsstoffe der Formel (IV)

Beispiel (IV-1)

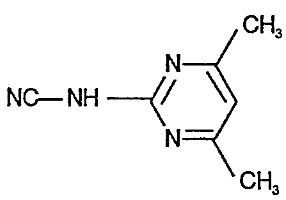

(Verfahren (b)

Ein Gemisch aus 42 g (0,5 Mol) Cyanoguanidin («Dicyandiamid») und 50 g (0,5 Mol) 2,4-Pentandion («Acetylaceton») wird 15 Stunden auf 120 °C erhitzt. Dann wird nach Abkühlen das Reaktionsgemisch mit 500 ml Wasser versetzt und die Lösung bei 0 °C bis 10 °C mit Salzsäure angesäuert. Das hierbei kristallin angefallene Produkt wird durch Absaugen isoliert.

Man erhält 51,8 g (70% der Theorie) 2-Cyanamino-4,6-dimethyl-pyrimidin vom Schmelzpunkt 205 °C.

Beispiel (IV-2)

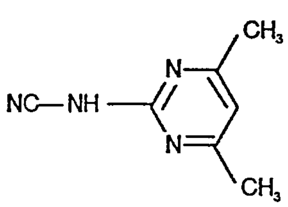

(Verfahren (c)

Eine auf 100 °C erhitzte Lösung von 24 g (0,427 Mol) Kaliumhydroxid in 100 ml Wasser wird bei 100 °C unter Rühren zu einer Mischung aus 9,2 g (0,043 Mol) N-(4,6-Dimethoxy-pyrimidin-2-yl)-thioharnstoff und 70 ml Wasser gegeben. Man rührt 2 Minuten bei 100 °C nach und gibt eine auf 100 °C erwärmte Lösung von 16,2 g (0,05 Mol) Blei(II)-acetat in 30 ml Wasser hinzu. Man erhitzt noch 5 Minuten unter Rückfluss, kühlt dann auf 0 °C bis 5 °C ab und versetzt die wässrige Lösung mit 30 ml Eisessig. Das hierbei kristallin ausfallende Produkt wird durch Absaugen isoliert.

Man erhält 6,3 g (81,5% der Theorie) 2-Cyanamino-4,6-dimethoxy-pyrimidin vom Schmelzpunkt 202 °C.

Zum gleichen Ergebnis kommt man, wenn man in Wasser- Methanol-Gemischen unter sonst gleichen Bedingungen arbeitet.

Nach den in den vorausgehenden Beispielen exemplarisch beschriebenen Verfahren können die in der nachstehenden Tabelle 3 aufgeführten Verbindungen der Formel (IV) hergestellt werden:

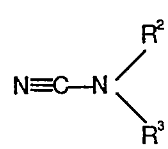 (IV)

Tabelle 3

| Beispiel-Nr. | $R^2$ | $R^3$ | Schmelzpunkt (°C) |
|---|---|---|---|
| IV-3 | H | 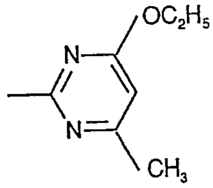 | |
| IV-4 | H | 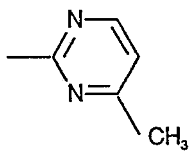 | 203 |
| IV-5 | H | 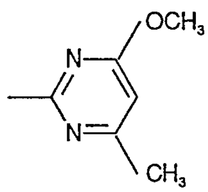 | 258 |
| IV-6 | H | 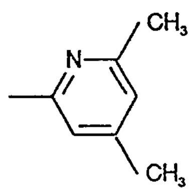 | 221 |
| IV-7 | H | 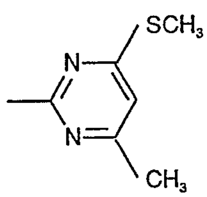 | |
| IV-8 | H | 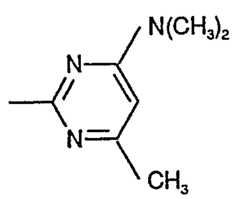 | |
| IV-9 | H | 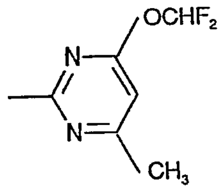 | 174 |

Tabelle 3 (Fortsetzung)

| Beispiel-Nr. | $R^2$ | $R^3$ | Schmelz-punkt (°C) |
|---|---|---|---|
| IV-10 | H | 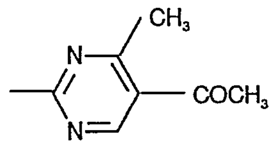 | 174 |
| IV-11 | H | 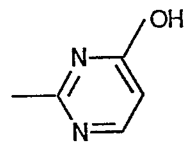 | > 300 |
| IV-12 | H | 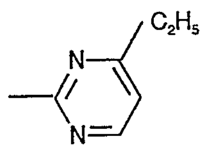 | 146 |
| IV-13 | H | 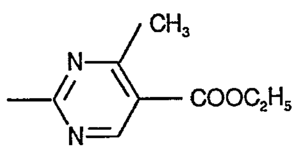 | 126 |
| IV-14 | H | 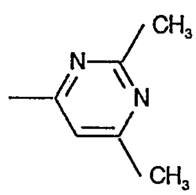 | 234 |
| IV-15 | H | 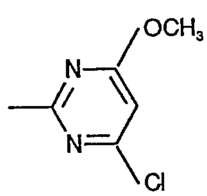 | 200 |
| IV-16 | H | 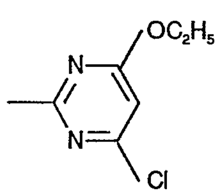 | |

Tabelle 3 (Fortsetzung)

| Beispiel-Nr. | $R^2$ | $R^3$ | Schmelzpunkt (°C) |
|---|---|---|---|
| IV-17 | H | 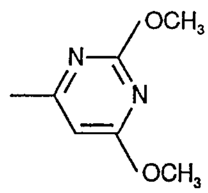 | |
| IV-18 | H | 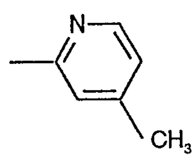 | 247-250 |
| IV-19 | H | 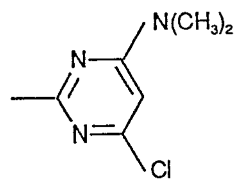 | |
| IV-20 | H | 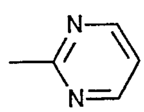 | 186 |
| IV-21 | H | 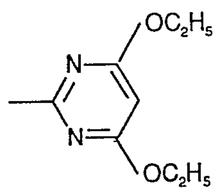 | |

2-(Alkyl-cyano-amino)-pyrimidine der Formel (IV) können beispielsweise wie folgt hergestellt werden:

Beispiel (IV-22)

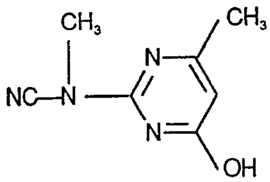

12,6 g (0,1 Mol) Dimethylsulfat werden zu einer Lösung von 15 g (0,1 Mol) 2-Cyanamino-4-hydroxy-6-methyl-pyrimidin - hergestellt nach Verfahren (b) - und 4,1 g (0,1 Mol) Natriumhydroxid in 60 ml Wasser tropfenweise gegeben, wobei die Reaktionstemperatur von 20°C auf 40°C steigt. Nach zweistündigem Rühren bei 20°C wird das kristallin angefallene Produkt durch Absaugen isoliert.

Man erhält 11,1 g (68% der Theorie) 2-(Methyl-cyano-amino)-4-hydroxy-6-methyl-pyrimidin vom Schmelzpunkt 290°C.

Analog erhält man:

Beispiel (IV-23)

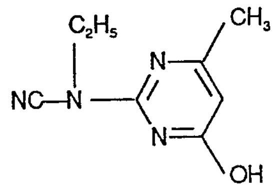

Fp. 215°C bis 220°C

Beispiel (IV-24)

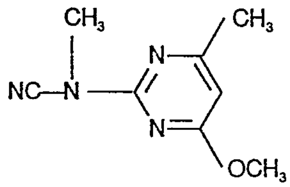

127,5 g (1 Mol) Dimethylsulfat werden zu einer Lösung von 75 g (0,5 Mol) 2-Cyanamino-4-hydroxy-6-methyl-pyrimidin - hergestellt nach Verfahren (b) - und 44 g (1,1 Mol) Natriumhydroxid in 750 ml Wasser tropfenweise gegeben, wobei die Reaktionstemperatur von 20 °C auf 35 °C ansteigt. Nach zwölfstündigem Rühren bei 20 °C wird durch Zugabe von Natronlauge ein pH-Wert zwischen 9 und 10 eingestellt und das kristallin angefallene Produkt durch Absaugen isoliert.

Man erhält 13 g (15% der Theorie) 2-(Methyl-cyano-amino)-4-methoxy-6-methyl-pyrimidin vom Schmelzpunkt 123 °C.

Analog erhält man:

Beispiel (IV-25)

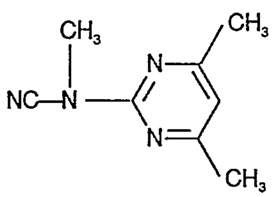

Fp. 104 °C

Beispiel (IV-26)

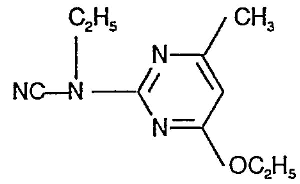

Fp. 71 °C

Herstellung der Ausgangsstoffe der Formel (X)

Beispiel (X-1)

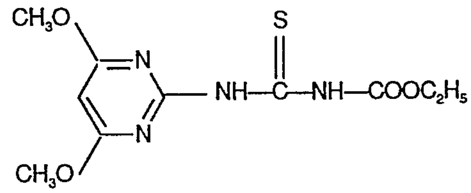

Eine Mischung aus 15,5 g (0,1 Mol) 2-Amino-4,6-dimethoxy-pyrimidin, 13,1 g (0,1 Mol) Ethoxycarbonyl-isothiocyanat und 200 ml Acetonitril wird 2 Stunden bei 60 °C gerührt. Dann wird auf 10 °C abgekühlt, und das kristallin angefallene Produkt wird durch Absaugen isoliert.

Man erhält 22,5 g (79% der Theorie) 1-(Ethoxycarbonyl)-3-(4,6-dimethoxy-pyrimidin-2-yl)-thioharnstoff vom Schmelzpunkt 194 °C (Zers.).

Nach dem im vorausgehenden Beispiel exemplarisch beschriebenen Verfahren können die in der nachstehenden Tabelle 4 aufgeführten Verbindungen der Formel (X) hergestellt werden:

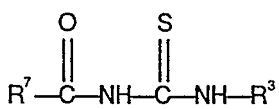 (X)

Tabelle 4

| Beisp.-Nr. | $R^7$ | $R^3$ | Schmelz-punkt (°C) |
|---|---|---|---|
| X-2 | 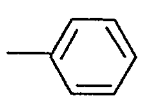 | 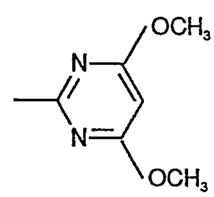 | 189 |
| X-3 | 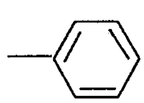 | 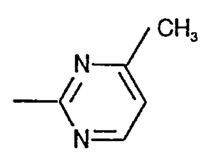 | 198-199 (Zers.) |

Tabelle 4 (Fortsetzung)

| Beisp.-Nr. | $R^7$ | $R^3$ | Schmelzpunkt (°C) |
|---|---|---|---|
| X-4 | $-OC_2H_5$ | 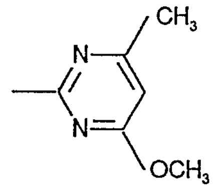 | 217 |
| X-5 | 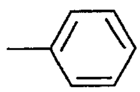 | 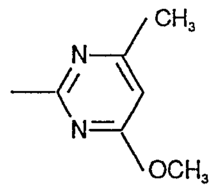 | 190 |
| X-6 | $-OC_2H_5$ | 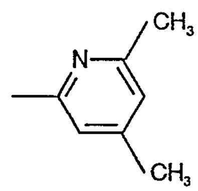 | 140 |
| X-7 | 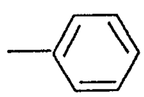 | 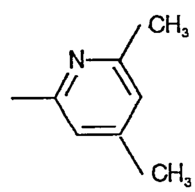 | 145 |
| X-8 | 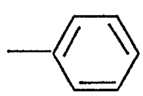 | 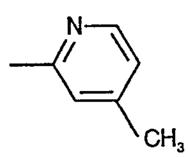 | 161 |
| X-9 | $-OC_2H_5$ | 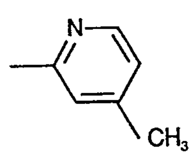 | 119 |
| X-10 | 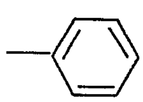 | 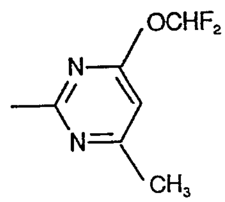 | 182 |

Tabelle 4 (Fortsetzung)

| Beisp.-Nr. | $R^7$ | $R^3$ | Schmelzpunkt (°C) |
|---|---|---|---|
| X-11 | $-OC_2H_5$ | 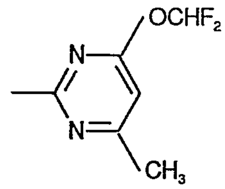 | 184-185 |
| X-12 | $-OC_2H_5$ | 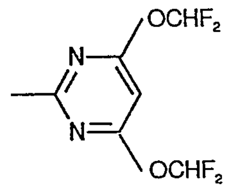 | 173 |
| X-13 | $-OC_2H_5$ | 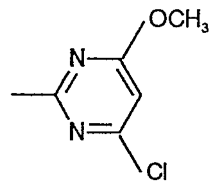 | 160-162 |
| X-14 | $-OC_2H_5$ | 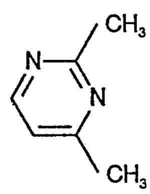 | 169 |
| X-15 | $-OC_2H_5$ | 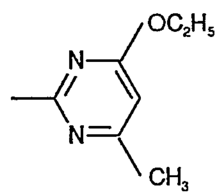 | |
| X-16 | $-OC_2H_5$ | 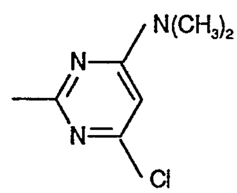 | 168 |
| X-17 | 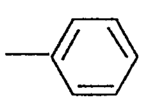 | 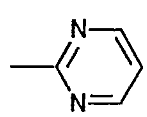 | 173 |

Tabelle 4 (Fortsetzung)

| Beisp.-Nr. | $R^7$ | $R^3$ | Schmelzpunkt (°C) |
|---|---|---|---|
| X-18 | 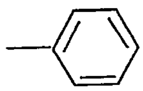 | 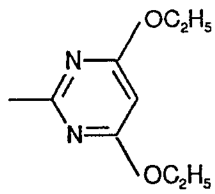 | 179 |
| X-19 | $-OC_2H_5$ | 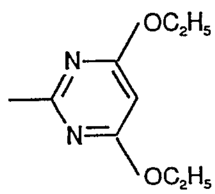 | 159 |
| X-20 | 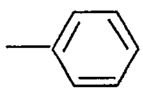 | 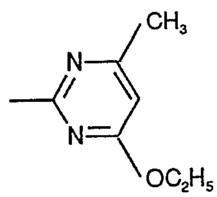 | 156 |

Herstellung der Ausgangsstoffe der Formel (XI)

Beispiel (XI-1)

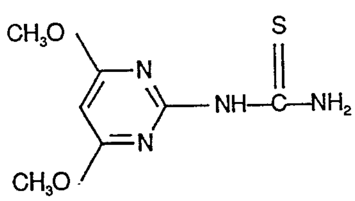

Eine Mischung aus 5,0 g (0,0175 Mol) 1-(Ethoxycarbonyl)-3-(4,6-dimethoxy-pyrimidin-2-yl)-thioharnstoff, 4,0 g (0,1 Mol) Natriumhydroxid und 100 ml Wasser wird 2 Tage bei 20 °C gerührt. Dann wird unter Rühren solange verdünnte Salzsäure zugetropft, bis die Lösung sauer gestellt ist und die $CO_2$-Entwicklung beendet ist. Das kristallin angefallene Produkt wird durch Absaugen isoliert.

Man erhält 3,5 g (94% der Theorie) 4,6-Dimethoxy-pyrimidin-2-yl-thioharnstoff vom Schmelzpunkt 245–248 °C (Zers.).

Nach dem im vorausgehenden Beispiel exemplarisch beschriebenen Verfahren können die in der nachstehenden Tabelle 5 aufgeführten Verbindungen der Formel (XI) hergestellt werden:

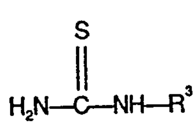 (XI)

Tabelle 5

| Beisp.-Nr. | $R^3$ | Schmelzpunkt (°C) |
|---|---|---|
| XI-2 | 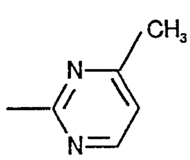 | 264–265 |

Tabelle 5 (Fortsetzung)

| Beisp.-Nr. | $R^3$ | Schmelzpunkt (°C) |
|---|---|---|
| XI-3 | 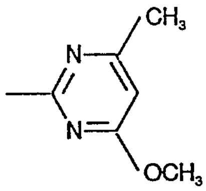 | 207 |
| XI-4 | 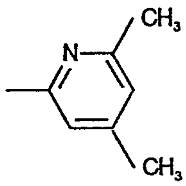 | 260 |
| XI-5 | 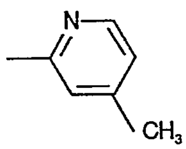 | 214–215 |
| XI-6 | 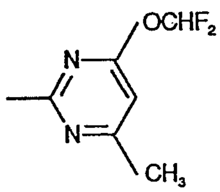 | 192–194 |
| XI-7 | 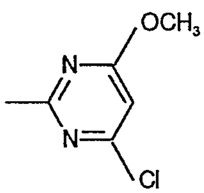 | 225–227 (Zers.) |
| XI-8 | 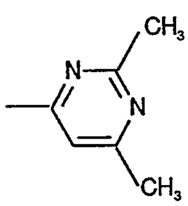 | 248 |
| XI-9 | 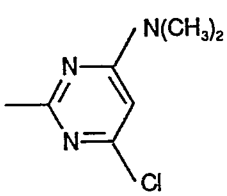 | |

Tabelle 5 (Fortsetzung)

| Beisp.-Nr. | $R^3$ | Schmelzpunkt (°C) |
| --- | --- | --- |
| XI-10 | 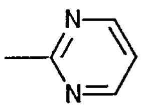 | 263 |

Beispiel A

Pre-emergence-Test/Gewächshaus

Lösungsmittel: Gewichtsteile Aceton

Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmässigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)

100% = totale Vernichtung

Bei diesem Test zeigte z.B. die folgende Verbindung der Herstellungsbeispiele (1) eine sehr gute Wirkung bei der Bekämpfung von mono- und dikotylen Unkräutern.

Beispiel B

Post-emergence-Test/Gewächshaus

Lösungsmittel: 5 Gewichtsteile Aceton

Emulgator: 1 Gewichtsteil Alkylarylpolyglykolether

Zur Herstellung einer zweckmässigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5–15 cm haben so, dass die jeweils gewünschten Wirkstoffmengen pro Flächeneinheit ausgebracht werden. Die Konzentration der Spritzbrühe wird so gewählt, dass in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)

100% = totale Vernichtung

Bei diesem Test zeigte z.B. die folgende Verbindung der Herstellungsbeispiele (1) eine sehr gute Wirksamkeit bei der Bekämpfung von mono- und dikotylen Unkräutern.

**Patentansprüche**

1. Benzodisultame der allgemeinen Formel (I)

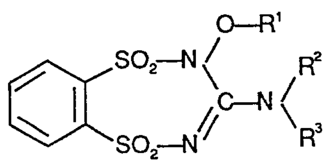

(I)

in welcher

$R^1$ für $C_1$–$C_{12}$-Alkyl [welches gegebenenfalls durch Fluor, Chlor, Cyano, $C_1$–$C_4$-Alkoxy, $C_1$–$C_4$-Alkylthio, $C_1$–$C_4$-Alkylsulfinyl, $C_1$–$C_4$-Alkylsulfonyl, $C_1$–$C_4$-Alkyl-carbonyl, $C_1$–$C_4$-Alkoxy-carbonyl, $C_1$–$C_4$-Alkylamino-carbonyl oder Di-($C_1$–$C_4$-alkyl)-amino-carbonyl substituiert ist], für $C_3$–$C_6$-Alkenyl (welches gegebenenfalls durch Fluor, Chlor oder Brom substituiert ist), $C_3$–$C_6$-Alkinyl, $C_3$–$C_6$-Cycloalkyl, $C_3$–$C_6$-Cycloalkyl-$C_1$–$C_2$-alkyl, Phenyl-$C_1$–$C_2$-alkyl (welches gegebenenfalls durch Fluor, Chlor, Nitro, Cyano, $C_1$–$C_4$-Alkyl, $C_1$–$C_4$-Alkoxy oder $C_1$–$C_4$-Alkoxycarbonyl substituiert ist) oder für Phenyl (welches gegebenenfalls durch Fluor, Chlor, Nitro, Cyano, $C_1$–$C_4$-Alkyl, Trifluormethyl, $C_1$–$C_4$-Alkoxy, $C_1$–$C_2$-Fluoralkoxy, $C_1$–$C_4$-Alkylthio, Trifluormethylthio oder $C_1$–$C_4$-Alkoxy-carbonyl substituiert ist) steht, in welcher weiter

$R^2$ für Wasserstoff, $C_1$–$C_4$-Alkyl [welches gegebenenfalls durch Fluor, Chlor, Cyano, $C_1$–$C_4$-Alkoxy, $C_1$–$C_4$-Alkylthio, $C_1$–$C_4$-Alkylsulfinyl, $C_1$–$C_4$-Alkylsulfonyl, $C_1$–$C_4$-Alkyl-carbonyl, $C_1$–$C_4$-Alkoxy-carbonyl, $C_1$–$C_4$-Alkylamino-carbonyl oder Di-($C_1$–$C_4$-alkyl)-amino-carbonyl substituiert ist], für $C_3$–$C_6$-Alkenyl, $C_3$–$C_6$-Alkinyl oderPhenyl-$C_1$–$C_2$-

alkyl (welches gegebenenfalls durch Fluor, Chlor, Nitro, Cyano, $C_1$-$C_4$-Alkyl, $C_1$-$C_4$-Alkoxy oder $C_1$-$C_4$-Alkoxycarbonyl substituiert ist) steht, in welcher ferner

$R^3$ für den Rest

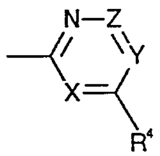

steht, worin

$R^4$ für Wasserstoff, Fluor, Chlor, Brom, Hydroxy, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), $C_1$-$C_4$-Alkylthio (welches gegebenenfalls durch Fluor und/oder Chlor substituiert ist), Amino, $C_1$-$C_4$-Alkylamino oder Di-($C_1$-$C_4$-alkyl)-amino steht,

X für Stickstoff oder eine Methin-brücke (CH) steht,

Y für Stickstoff oder eine gegebenenfalls substituierte Methin-brücke C-$R^5$ steht, wobei

$R^5$ für Wasserstoff, Fluor, Chlor, Brom, $C_1$-$C_4$-Alkyl, $C_1$-$C_4$-Alkylcarbonyl oder $C_1$-$C_4$-Alkoxycarbonyl steht und

Z für Stickstoff oder eine gegebenenfalls substituierte Methin-brücke C-$R^6$ steht, wobei

$R^6$ für Wasserstoff, Fluor, Chlor, Brom, Hydroxy, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), $C_1$-$C_4$-Alkylthio (welches gegebenenfalls durch Fluor und/oder Chlor substituiert ist), Amino, $C_1$-$C_4$-Alkylamino oder Di-($C_1$-$C_4$-alkyl)-amino steht.

2. Verbindungen der allgemeinen Formel (I) gemäss Anspruch 1, dadurch gekennzeichnet, dass darin

$R^1$ für $C_1$-$C_8$-Alkyl (welches gegebenenfalls durch Fluor oder Chlor substituiert ist), $C_3$-$C_4$-Alkenyl, $C_1$-$C_2$-Alkoxy-carbonylmethyl, Phenyl, Phenylethyl oder Benzyl (welches gegebenenfalls durch Fluor, Chlor, Nitro, Cyano, Methyl, Methoxy oder Methoxy-carbonyl substituiert ist) steht,

$R^2$ für Wasserstoff steht und

$R^3$ für den Rest

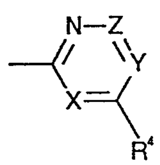

steht, worin

$R^4$ für Chlor, Methyl, Ethyl, Methoxy, Ethoxy, Methylthio oder Difluormethoxy steht,

X für Stickstoff steht,

Y für eine Methin-brücke (CH) steht, und

Z für eine gegebenenfalls substituierte Methin-brücke C-$R^6$ steht, wobei

$R^6$ für Wasserstoff, Chlor, Methyl, Methoxy, Ethoxy, Methylthio, Ethylthio, Dimethylamino oder Diethylamino steht.

3. Verfahren zur Herstellung von Benzodisultamen der allgemeinen Formel (I) gemäss Anspruch 1, dadurch gekennzeichnet, dass man Benzol-1,2-disulfonsäuredichlorid der Formel (II)

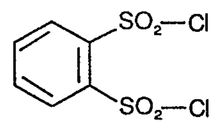

(II)

mit Oxyguanidin-Derivaten der Formel (III)

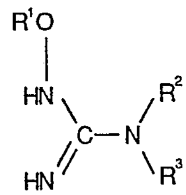

(III)

in welcher

$R^1$, $R^2$ und $R^3$ die im Anspruch 1 angegebenen Bedeutungen haben, in Gegenwart von Säureakzeptoren und gegebenenfalls in Gegenwart von Verdünnungsmitteln umsetzt.

4. Herbizide Mittel, gekennzeichnet durch einen Gehalt an mindestens einem Benzodisultam der allgemeinen Formel (I) gemäss Anspruch 1.

5. Verwendung von Benzodisultamen der allgemeinen Formel (I) gemäss Anspruch 1 zur Bekämpfung von unerwünschtem Pflanzenwachstum.

6. Verfahren zur Herstellung von herbiziden Mitteln, dadurch gekennzeichnet, dass man Benzodisultame der allgemeinen Formel (I) gemäss Anspruch 1 mit Streckmitteln und/oder oberflächenaktiven Mitteln vermischt.

**Claims**

1. Benzodisultams of the general formula (I)

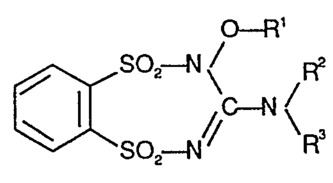

(I)

in which

$R^1$ represents $C_1$-$C_{12}$-alkyl [which is optionally substituted by fluorine, chlorine, cyano, $C_1$-$C_4$-alkoxy, $C_1$-$C_4$-alkylthio, $C_1$-$C_4$-alkylsulphinyl, $C_1$-$C_4$-alkylsulphonyl, $C_1$-$C_4$-alkyl-carbonyl, $C_1$-$C_4$-alkoxy-carbonyl, $C_1$-$C_4$-alkyl-amino-carbonyl or di-($C_1$-$C_4$-alkyl)-amino-carbonyl], or represents $C_3$-$C_6$-alkenyl (which is optionally substituted by

fluorine, chlorine or bromine), $C_3$-$C_6$-alkinyl, $C_3$-$C_6$-cycloalkyl, $C_3$-$C_6$-cycloalkyl-$C_1$-$C_2$-alkyl or phenyl-$C_1$-$C_2$-alkyl (which is optionally substituted by fluorine, chlorine, nitro, cyano, $C_1$-$C_4$-alkyl, $C_1$-$C_4$-alkoxy or $C_1$-$C_4$-alkoxycarbonyl), or represents phenyl (which is optionally substituted by fluorine, chlorine, nitro, cyano, $C_1$-$C_4$-alkyl, trifluoromethyl, $C_1$-$C_4$-alkoxy, $C_1$-$C_2$-fluoroalkoxy, $C_1$-$C_4$-alkylthio, trifluoromethylthio or $C_1$-$C_4$-alkoxy-carbonyl), in which, furthermore,

$R^2$ represents hydrogen, $C_1$-$C_4$-alkyl [which is optionally substituted by fluorine, chlorine, cyano, $C_1$-$C_4$-alkoxy, $C_1$-$C_4$-alkylthio, $C_1$-$C_4$-alkylsulphinyl, $C_1$-$C_4$-alkylsulphonyl, $C_1$-$C_4$-alkylcarbonyl, $C_1$-$C_4$-alkoxy-carbonyl, $C_1$-$C_4$-alkylamino-carbonyl or di-($C_1$-$C_4$-alkyl)-amino-carbonyl], or represents $C_3$-$C_6$-alkenyl, $C_3$-$C_6$-alkinyl or phenyl-$C_1$-$C_2$-alkyl (which is optionally substituted by fluorine, chlorine, nitro, cyano, $C_1$-$C_4$-alkyl, $C_1$-$C_4$-alkoxy or $C_1$-$C_4$-alkoxycarbonyl), in which, furthermore,

$R^3$ represents the radical formula

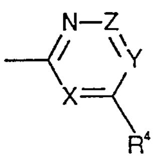

wherein

$R^4$ represents hydrogen, fluorine, chlorine, bromine, hydroxyl, $C_1$-$C_4$-alkyl (which is optionally substituted by fluorine and/or chlorine), $C_1$-$C_4$-alkoxy (which is optionally substituted by fluorine and/or chlorine), $C_1$-$C_4$-alkylthio (which is optionally substituted by fluorine and/or chlorine), amino, $C_1$-$C_4$-alkylamino or di-($C_1$-$C_4$-alkyl)-amino,

X represents nitrogen or a methine bridge (CH),

Y represents nitrogen or an optionally substituted methine bridge C-$R^5$, wherein

$R^5$ represents hydrogen, fluorine, chlorine, bromine, $C_1$-$C_4$-alkyl, $C_1$-$C_4$-alkylcarbonyl or $C_1$-$C_4$-alkoxycarbonyl and

Z represents nitrogen or an optionally substituted methine bridge C-$R^6$, wherein

$R^6$ represents hydrogen, fluorine, chlorine, bromine, hydroxyl, $C_1$-$C_4$-alkyl (which is optionally substituted by fluorine and/or chlorine), $C_1$-$C_4$-alkoxy (which is optionally substituted by fluorine and/or chlorine), $C_1$-$C_4$-alkylthio (which is optionally sustituted by fluorine and/or chlorine), amino, $C_1$-$C_4$-alkylamino or di-($C_1$-$C_4$-alkyl)-amino.

2. Compounds of the general formula (I) according to Claim 1, characterized in that, in this formula,

$R^1$ represents $C_1$-$C_8$-alkyl (which is optionally substituted by fluorine or chlorine), $C_3$-$C_4$-alkenyl, $C_1$-$C_2$-alkoxy-carbonylmethyl, phenyl, phenethyl or benzyl (which is optionally substituted by fluorine, chlorine, nitro, cyano, methyl, methoxy or methoxy-carbonyl),

$R^2$ represents hydrogen and

$R^3$ represents the radical formula

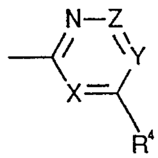

wherein

$R^4$ represents chlorine, methyl, ethyl, methoxy, ethoxy, methylthio or difluoromethoxy,

X represents nitrogen,

Y represents a methine bridge (CH) and

Z represents an optionally substituted methine bridge C-$R^6$, wherein

$R^6$ represents hydrogen, chlorine, methyl, methoxy, ethoxy, methylthio, ethylthio, dimethylamino or diethylamino.

3. Process for the preparation of benzodisultams of the general formula (I) according to Claim 1, characterized in that benzene-1,2-disulphonic acid dichloride of the formula (II)

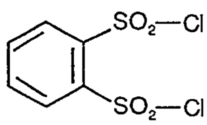

(II)

are reacted with oxyguanidine derivatives of the formula (III)

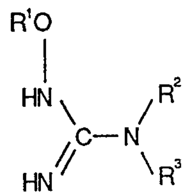

(III)

in which

$R^1$, $R^2$ and $R^3$ have the meanings given in Claim 1, in the presence of acid acceptors and if appropriate in the presence of diluents.

4. Herbicidal agents, characterized in that they contain at least one benzodisultam of the general formula (I) according to Claim 1.

5. Use of benzodisultams of the general formula (I) according to Claim 1 for combating undesirable plant growth.

6. Process for the preparation of herbicidal agents, characterized in that benzodisultams of the general formula (I) according to Claim 1 are mixed with extenders and/or surface-active agents.

**Revendications**

1. Benzodisultames de formule générale (I)

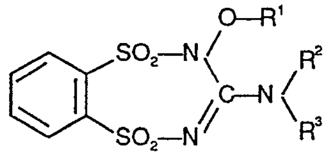

(I)

dans laquelle

$R^1$ représente un groupe alkyle en $C_1$–$C_{12}$ [éventuellement substitué par du fluor, du chlore, un groupe cyano, un groupe alcoxy en $C_1$–$C_4$, un groupe alkylthio en $C_1$–$C_4$, un groupe alkylsulfinyle en $C_1$–$C_4$, un groupe alkylsulfonyle en $C_1$–$C_4$, un groupe alkyl en $C_1$–$C_4$-carbonyle, un groupe alcoxy en $C_1$–$C_4$-carbonyle, un groupe alkyl en $C_1$–$C_4$-amino-carbonyle ou un groupe di-(alkyl en $C_1$–$C_4$)-aminocarbonyle], représente un groupe alcényle en $C_3$–$C_6$ (éventuellement substitué par du fluor, du chlore ou du brome), un groupe alcynyle en $C_3$–$C_6$, cyclo-alkyle en $C_3$–$C_6$, cycloalkyl en $C_3$–$C_6$-alkyle en $C_1$–$C_2$, phényl-alkyle en $C_1$–$C_2$ (éventuellement substitué par du fluor, du chlore, un groupe nitro, un groupe cyano, un groupe alkyle en $C_1$–$C_4$, alcoxy en $C_1$–$C_4$ ou alcoxy en$C_1$–$C_4$-carbonyle) ou représente le radical phényle (éventuellement substitué par du fluor, du chlore, un groupe nitro, un groupe cyano, un groupe alkyle en $C_1$–$C_4$, trifluorométhyle, alcoxy en $C_1$–$C_4$, fluoro-alcoxy en $C_1$–$C_2$, alkylthio en $C_1$–$C_4$, trifluorométhylthio ou alcoxy en $C_1$–$C_4$-carbonyle), et dans laquelle

$R^2$ représente l'hydrogène, un groupe alkyle en $C_1$–$C_4$ [éventuellement substitué par du fluor, du chlore, un groupe cyano, un groupe alcoxy en $C_1$–$C_4$, un groupe alkylthio en $C_1$–$C_4$, un groupe alkylsulfinyle en $C_1$–$C_4$, un groupe alkylsulfonyle en $C_1$–$C_4$, un groupe alkyl en $C_1$–$C_4$-carbonyle, un groupe alcoxy en $C_1$–$C_4$-carbonyle, un groupe alkyl en $C_1$–$C_4$-amino-carbonyle ou un groupe (dialkyl en $C_1$–$C_4$)-amino-carbonyle], représente un groupe alcényle en $C_3$–$C_6$, alcynyle en $C_3$–$C_6$ ou phényl-alkyle en $C_1$–$C_2$ (éventuellement substitué par du fluor, du chlore, un groupe nitro, cyano, alkyle en $C_1$–$C_4$, alcoxy en $C_1$–$C_4$ ou alcoxy en $C_1$–$C_4$-carbonyle), et dans laquelle

$R^3$ représente le reste

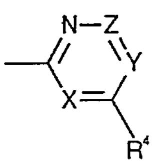

dans lequel

$R^4$ représente l'hydrogène, le fluor, le chlore, le brome, un groupe hydroxy, un groupe alkyle en $C_1$–$C_4$ (éventuellement substitué par du fluor et/ou du chlore), alcoxy en $C_1$–$C_4$ (éventuellement substitué par du fluor et/ou du chlore), alkylthio en $C_1$–$C_4$ (éventuellement substitué par du fluor et/ou du chlore), un groupe amino, un groupe alkylamino en $C_1$–$C_4$ ou di-(alkyl en $C_1$–$C_4$)-amino,

X représente l'azote ou un pont méthine (CH),

Y représente l'azote ou un pont méthine C–$R^5$ éventuellement substitué, dans lequel

$R^5$ représente un atome d'hydrogène, de fluor, de chlore, de brome, un groupe alkyle en $C_1$–$C_4$, alkyl en $C_1$–$C_4$-carbonyle ou alcoxy en $C_1$–$C_4$ carbonyle, et

Z représente l'azote ou un pont méthine C–$R^6$ éventuellement substitué, dans lequel

$R^6$ représente un atome d'hydrogène, de fluor, de chlore, de brome, un groupe alkyle en $C_1$–$C_4$ (éventuellement substitué par du fluor et/ou du chlore), alcoxy en $C_1$–$C_4$ (éventuellement substitué par du fluor et/ou du chlore), alkylthio en $C_1$–$C_4$ (éventuellement substitué par du fluor et/ou du chlore), un groupe amino, alkylamino en $C_1$–$C_4$ ou di-(alkyl en $C_1$–$C_4$)-amino.

2. Composés de formule générale (I) selon la revendication 1, caractérisé en ce que, dans cette formule:

$R^1$ représente un groupe alkyle en $C_1$–$C_8$ (éventuellement substitué par du fluor ou du chlore), un groupe alcényle en $C_3$–$C_4$, alcoxy en $C_1$–$C_2$-carbonyl-méthyle, phényle, phényléthyle ou benzyle (éventuellement substitué par du fluor, du chlore, un groupe nitro, un groupe cyano, méthyle, méthoxy ou méthoxy-carbonyle),

$R^2$ représente l'hydrogène et

$R^3$ représente le reste

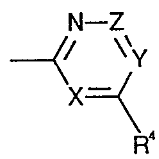

dans lequel

$R^4$ représente un atome de chlore, un groupe méthyle, éthyle, méthoxy, éthoxy, méthylthio ou difluorométhoxy,

X représente l'azote,

Y représente un pont méthine (CH), et

Z représente un pont méthine C–$R^6$ éventuellement substitué, dans lequel

$R^6$ représente l'hydrogène, le chlore, un groupe méthyle, méthoxy, éthoxy, méthylthio, éthylthio, diméthylamino ou diéthylamino.

3. Procédé pour la fabrication des benzodisultames de formule générale (I) selon la revendication 1, caractérisé en ce que l'on fait réagir du dichlorure d'acide benzène-1,2-disulfonique de formule (II)

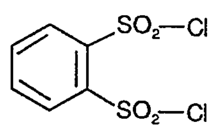

(II)

avec des dérivés d'oxyguanidine de formule (III)

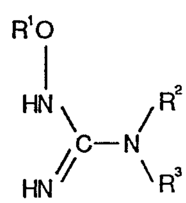

(III)

dans laquelle

$R^1$, $R^2$ et $R^3$ ont les significations décrites dans la revendication 1, en présence d'accepteurs d'acides et éventuellement en présence de diluants.

4. Agents herbicides caractérisés en ce qu'ils contiennent au moins un benzodisultame de formule générale (I) selon la revendication 1.

5. Utilisation des benzodisultames de formule générale (I) selon la revendication 1 pour la lutte contre la croissance de plantes indésirables.

6. Procédé pour la fabrication d'agents herbicides, caractérisé en ce que l'on mélange les benzodisultames de formule générale (I) selon la revendication 1 avec des agents de dilution et/ou des agents tensio-actifs.